# EUROPEAN PATENT APPLICATION

(11) **EP 4 292 591 A1**
(43) Date of publication of application: **20.12.2023**
(21) Application number: 22179045.4
(22) Date of filing: 14.06.2022
(51) Int. Cl.: A61K 31/18, A61P 13/12

(54) **COMPOUNDS FOR USE IN THE TREATMENT OF KIDNEY DISEASES**

(71) Applicant: Universitätsmedizin Greifswald, 17475 Greifswald (DE)
(72) Inventor: Endlich, Nicole, 17493 Greifswald (DE); Schindler, Maximilian, 17489 Greifswald (DE)
(74) Representative: Cohausz & Florack

(57) **Abstract**

The present invention relates to a compound for use in the treatment and/or prevention of a kidney disease, wherein the compound has the Formula 1, or a salt, hydrate, solvate, metabolite, or prodrug thereof.

## Description

### FIELD OF THE INVENTION

The present invention relates to compounds for use in the treatment of a kidney disease. The compounds can be particularly used in the treatment and/or prevention of a kidney disease.

### BACKGROUND OF THE INVENTION

A global prevalence of more than 10% makes chronic kidney diseases a silent pandemic which causes severe and life-shortening problems for patients as well as the health care systems worldwide. In more than 75% of the kidney patients, a highly specific cell type in the kidney, the podocytes, is damaged or lost. Since podocytes are post-mitotic, there is no possibility of regeneration and lost podocytes are lost forever.

Podocytes cover the outer aspect of the capillaries in the filter unit of the kidney, the glomeruli, and are an essential part of the filtration barrier. Their tiny cell processes, so called foot processes (FP), interdigitate in a very regular and zipper-like manner. Between of the interdigitating foot processes, a slit membrane is spanned which is also essential for the size selectivity of the filtration barrier (Pavenstadt, H. et al. "Cell biology of the glomerular podocyte." Physiological reviews 83.1 (2003): 253-307; Garg, Puneet. "A review of podocyte biology." American journal of nephrology 47.1 (2018): 3-13). Injury of FP or detachment of podocytes leads to proteinuria, which is a clinical hallmark of kidney disease and characterized by the loss of high molecular weight proteins.

Such a damage to podocyte foot processes often leads to end-stage renal disease (ESRD, Shankland, S. J. "The podocyte's response to injury: role in proteinuria and glomerulosclerosis." Kidney international 69.12 (2006): 2131-2147; Kriz, Wilhelm. "Podocyte hypertrophy mismatch and glomerular disease." Nature Reviews Nephrology 8.11 (2012): 618-619) and finally to renal failure making dialysis and transplantation necessary to survive. One specific podocytopathy is the focal and segmental glomerulosclerosis (FSGS). FSGS is characterized by the effacement of FP, the loss of podocytes, matrix accumulation, activated parietal epithelial cell (PECs) of the Bowman's capsule and sclerotic lesions in the glomeruli. Since many kidney disease are painless, kidney diseases, in particular glomerulopathies and/or podocyte-injury-related diseases, are diagnosed very late. Moreover, no healing drugs or therapies are available. Thus, there is a need for improved methods to treat and prevent kidney diseases.

### SUMMARY OF THE INVENTION

Against the aforementioned background, it is an object of the present invention to provide improved methods of treatment and prevention of kidney diseases, in particular to provide safe and effective methods for treatment and prevention of acute and chronic kidney diseases.

These objects are achieved by the compounds for use of claim 1. In particular, the invention provides a compound for use in the treatment and/or prevention of a kidney disease, wherein the compound has the formula 1: 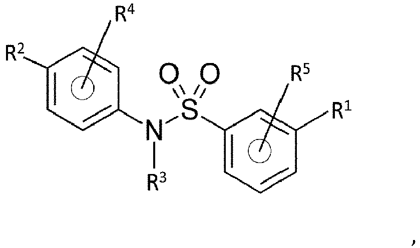
or a salt, hydrate, solvate, metabolite or prodrug thereof;
wherein R₁ is selected from the group consisting of hydrogen, -CH=CH-CH(O)-NH-OH, - (CH₂)₂-C(O)NHOH, or -C≡C-CH(O)NHOH;
R2 is selected from the group consisting of hydrogen, (CH₂)₂-CH₂ and HC=C-CH=CH-CH(O)NHOH;
R₃ is selected from the group consisting of H and C₁-C₆-alkyl; and
R₄ and R₅ are independently selected from the group consisting of hydrogen, halogen, C₁-C₆ alkyl, CN, and CF₃.

The inventors found that compounds of formula 1 were particularly effective in a well-established animal model of focal and segmental glomerulosclerosis (FSGS), a kidney disease and in particular a disease related to podocyte injury.

By employing the compounds of Formula 1 (or salts, solvates, hydrates, metabolites, or prodrugs thereof) in the treatment of kidney diseases, in particular of chronic or acute kidney diseases, the inventors provide a greatly needed means to safely and effectively treat and prevent podocyte damage, apoptosis, or loss, which is a hallmark of kidney diseases, in particular of podocyte injury-related diseases such as FSGS and minimal change disease.

In particular, the inventors found that in the well-established zebrafish larvae model of FSGS, where apoptosis of podocytes is induced in a gradual and specific manner (Hansen, Kerrin Ursula Ingeborg, et al. "Prolonged podocyte depletion in larval zebrafish resembles mammalian focal and segmental glomerulosclerosis." The FASEB Journal 34.12 (2020): 15961-15974), two test compounds, (2E)-N-hydroxy-3-[3-(phenylsulfamoyl)phenyl]prop-2-enamide (also known as Belinostat^{®} or Beleodaq) and 5-[3-(benzenesulfonamido)phenyl]-N-hydroxypent-2-en-4-ynamide (also known as oxamflatin) significantly slowed the progression of the podocyte injury induced in the disease model and even improved disease-related symptoms. The effects occurred in a concentration-dependent manner.

Thus, the invention provides safe and effective compounds for use in treating and/or preventing kidney diseases, in particular of kidney diseases such as glomerulopathies and/or podocyte injury-related diseases such as FSGS and minimal change disorder.

### DETAILED DESCRIPTION OF ILLUSTRATIVE EMBODIMENTS

The invention provides compounds for use in the treatment and/or prevention of a kidney disease, wherein the compound has the formula 1: 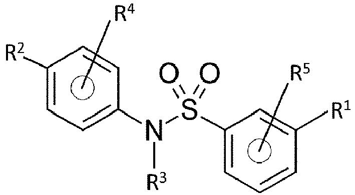 ,
or a salt, hydrate, solvate, metabolite, or prodrug thereof;
wherein R₁ is selected from the group consisting of hydrogen, CH=CH-CH(O)NHOH, (CH₂)₂-C(O)NHOH, and C≡C-CH(O)NHOH;
R₂ is selected from the group consisting of hydrogen, (CH₂)₂-CH₂ and C=C-CH=CH-CH(O)NHOH;
R₃ is selected from the group consisting of H and C₁-C₆-alkyl; and
R₄ and R₅ are independently selected from the group consisting of hydrogen, halogen, C₁-C₆ alkyl, CN, and CF₃.

The inventors found that compounds with the shared general structure of Formula 1 as described above had a protective effect on podocytes in a validated injury model of focal and segmental glomerulosclerosis (FSGS). This FSGS model is also representative of other kidney diseases, in particular acute and chronic kidney diseases, including but not limited to other glomerulopathies and podocyte-injury-related diseases.

### Compounds

The compound for use of the invention has the general structure of formula 1: 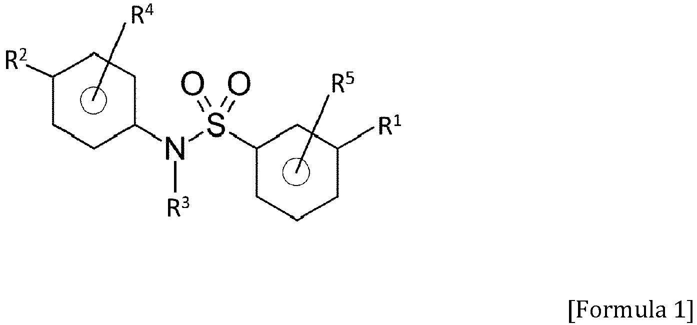
wherein R₁ is selected from the group consisting of hydrogen, -CH=CH-CH(O)-NH-OH, - (CH₂)₂-C(O)NHOH, or -C=C-CH(O)NHOH;
R₂ is selected from the group consisting of hydrogen, (CH₂)₂-CH₂, and C=C-CH=CH-CH(O)NHOH;
R₃ is selected from the group consisting of H and C₁-C₆-alkyl; and
R₄ and R₅ are independently selected from the group consisting of hydrogen, halogen, C₁-C₆ alkyl, CN, and CF₃.

The compound for use of the invention may also be a pharmaceutically-acceptable salt, solvate, or hydrate of Formula 1 described above. As used herein, the terms "salt" and pharmaceutically acceptable salt" are used interchangeably. Examples of pharmaceutically acceptable salts are well known in the art and are for example discussed in Berge et at., 1977, "Pharmaceutically Acceptable Salts." J. Pharm. ScL. vol. 66, pp. 1-19. In some embodiments, the pharmaceutically-acceptable salt is selected from the group of inorganic cations, organic cations, inorganic anions, organic anions, and polymeric organic anions.

Examples of suitable inorganic cations include, but are not limited to, alkali metal ions such as Na⁺ and K+, alkaline earth cations such as Ca²⁺ and Mg²⁺, and other cations such as Al+3 . Examples of suitable organic cations include, but are not limited to, ammonium ion (i.e., NH4 + ) and substituted ammonium ions (e.g., NH3 R+, NH2 R2 +, NHR3 +, NR4 + ). Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH3)4+. Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyoxybenzoic, acetic, ascorbic, aspartic, benzoic, camphorsulfonic, cinnamic, citric, edetic, ethanedisulfonic, ethanesulfonic, fumaric, glucheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric.

Examples of suitable polymeric organic anions include, but are not limited to, those derived from the following polymeric acids: tannic acid, carboxymethyl cellulose.

Representative salts include the following salts: acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, pamoate (embonate), palmitate, pantothenate, phosphate/diphosphate, polygalacturonate, potassium, salicylate, sodium, stearate, subacetate, succinate, tannate, tartrate, teoclate, tosylate, triethiodide, trimethylammonium and valerate.

The terms "solvate" and "hydrate" have their normal meaning in the art. The term "solvate", for example, is used herein in the conventional sense to refer to a complex of solute and solvent. If the solvent is water, the solvate may be conveniently referred to as a hydrate, for example, a mono-hydrate, a di-hydrate, a tri-hydrate.

The compound for use according to the invention in some embodiments is a produg of the compound of Formula 1. A prodrug has its normal meaning in the art and refers to a pharmacologically inactive drug precursor that is converted into a pharmacologically active drug compound.

In a particularly preferred embodiment, the compound for use has the structure of Formula 1, wherein R₁ is CH=CH-C(O)NHOH and R₂₋₅ are H, in particular wherein R₁ is (*E*)-CH=CH-CCO)NHOH and R₂₋₅ are H. In a particularly preferred embodiment, the compound for use has the structure of Formula 2:

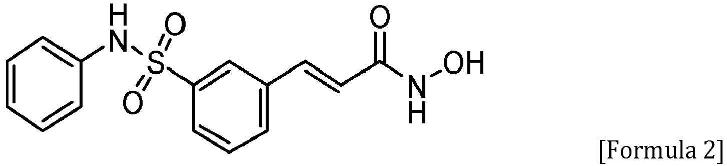

This corresponds to (2E)-N-hydroxy-3-[3-(phenylsulfamoyl)phenyl]prop-2-enamide, also known as Beleodaq or Belinostat^{™}, previously known as PXD101 or PX 105684, is a well-known histone deacetylase inhibitor with a sulfonamide-hydroxamide structure. The molecular formula is C₁sH₁₄N₂O₄S and the molecular weight is 318.35 g/mol. It has been studied for its efficacy in treating tumors, e.g. various solid-tumours (see Steele et al., 2008) and has been approved for the treatment of peripheral T-cell lymphoma. It's approved pharmaceutical formulation is intravenous administration.

Thus, in a particularly preferred embodiment, the compound for use is (2E)-N-hydroxy-3-[3-(phenylsulfamoyl)phenyl]prop-2-enamide (i.e. Formula 2-5 shown above, corresponding to belinostat), or a salt, hydrate, solvate, or metabolite thereof.

In some embodiments, the compound for use comprises or consists of a metabolite of belinostat, preferably selected from the group consisting of belinostat amide, belinostat acid, methyl belinostat, belinostat gluconoride, 3-(anilinosulfonyl)-benzenecarboxylic acid (3-ASBA), and belinostatglucoside. Metabolites of belinostat preferably are selected from the group consisting of the following structures:

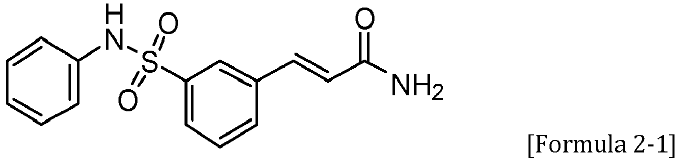

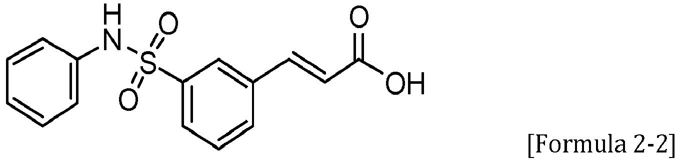

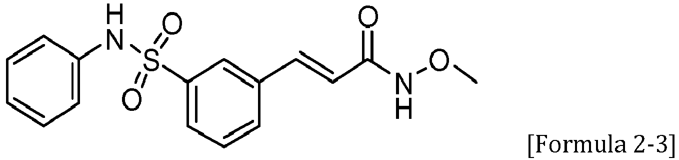

In some embodiments, the compound for use of the invention comprises or consists of a prodrug of belinostat. The prodrug of belinostat is preferably selected from the group consisting of ZL277 (Formula 2-4), ZL277-B(OH)₂-452 (Formula 2-5), and ZL277-OH-424 (Formula 2-6)(Zhang et al., (2019) Pharmaceuticals, 12(4), 180), which have the following structures:

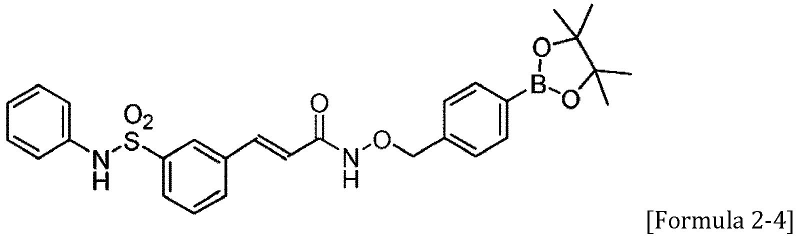

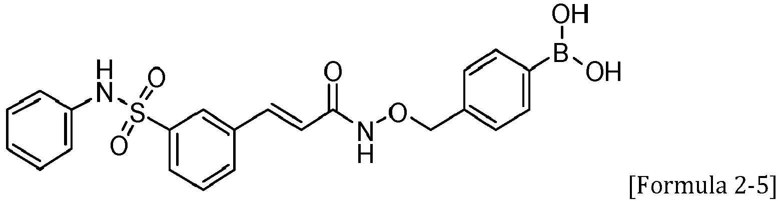

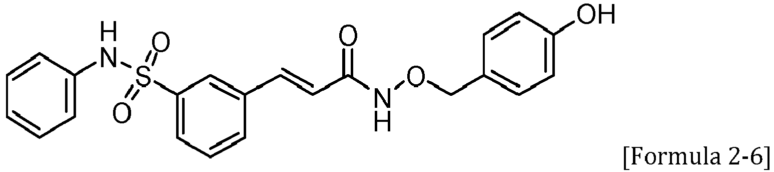

In another preferred embodiment, the compound for use of the invention is an analog of Formula 1, in particular an analog of belinostat (Formula 2) or of a metabolite (e.g. Formula 2-1 to 2-3) or prodrug (Formula 2-4 to 2-6) of belinostat. In a particularly preferred embodiment, the compound for use of the invention is an analog of belinostat (Formula 2), in particular one selected from the group consisting of (E)-3-(3-(hydroxyamino)-3-oxoprop-1-en-1-yl)-N-phenylbenzamide, (E)-3-(3-(hydroxyamino)-3-oxoprop-1-en-1-yl)-N-(o-tolyl)benzamide, (E)-3-(3-(hydroxyamino)-3-oxoprop-1-en-1-yl)-N-(m-tolyl)benzamide, (E)-3-(3-(hydroxyamino)-3-oxoprop-1-en-1-yl)-N-(p-tolyl)benzamide, (E)-3-(3-(hydroxyamino)-3-oxoprop-1-en-1-yl)-N-(2-methoxyphenyl)benzamide, (E)-3-(3-(hydroxyamino)-3-oxoprop-1-en-1-yl)-N-(3-methoxyphenyl)benzamide, (E)-3-(3-(hydroxyamino)-3-oxoprop-1-en-1-yl)-N-(4-methoxyphenyl)benzamide, (E)-N-(2-aminophenyl)-3-(3-(hydroxyamino)-3-oxoprop-1-en-1-yl)benzamide, (E)-N-(4-(dimethylamino)phenyl)-3-(3-(hydroxyamino)-3-oxoprop-1-en-1-yl)benzamide, (E)-N-(2-bromophenyl)-3-(3-(hydroxyamino)-3-oxoprop-1-en-1-yl)benzamide, (E)-N-(3-bromophenyl)-3-(3-(hydroxyamino)-3-oxoprop-1-en-1-yl)benzamide, (E)-N-(4-bromophenyl)-3-(3-(hydroxyamino)-3-oxoprop-1-en-1-yl)benzamide, (E)-N-(2-fluorophenyl)-3-(3-(hydroxyamino)-3-oxoprop-1-en-1-yl)benzamide, (E)-N-(4-chlorophenyl)-3-(3-(hydroxyamino)-3-oxoprop-1-en-1-yl)benzamide, (E)-3-(3-(hydroxyamino)-3-oxoprop-1-en-1-yl)-N-(pyridin-2-yl)benzamide, (E)-3-(3-(hydroxyamino)-3-oxoprop-1-en-1-yl)-N-(thiazol-2-yl)benzamide, (E)-3-(3-(4-aminophenoxy)phenyl)-N-hydroxyacrylamide, (E)-N-hydroxy-3-(3-(4-(methylamino)phenoxy)phenyl)acrylamide, and (E)-3-(3-(4-(dimethylamino)phenoxy)phenyl)-N-hydroxyacrylamide.

In another preferred embodiment, the compound for use of the invention has the structure of Formula 1, wherein R₁ and R₃₋₅ are H, and wherein R₂ is C=C-CH=CH-C(O)NHOH, in particular wherein C≡C-(*E*)-CH=CH-C(O)NHOH. In a preferred embodiment, the invention provides 5-[3-(benzenesulfonamido)phenyl]-N-hydroxypent-2-en-4-ynamide (i.e. Formula 3 shown below, or oxamflatin), or a salt, hydrate, solvate, or metabolite thereof, for use in a method of treatment of a kidney disease.

5-[3-(benzenesulfonamido)phenyl]-N-hydroxypent-2-en-4-ynamide, also known as oxamflatin or Metacept 3 (CAS#: 151720-43-3) is a sulfonamide that is known for its role as a histone deacetylase inhibitor and has been studied for its role in various cancers. Oxamflatin has the following structure:

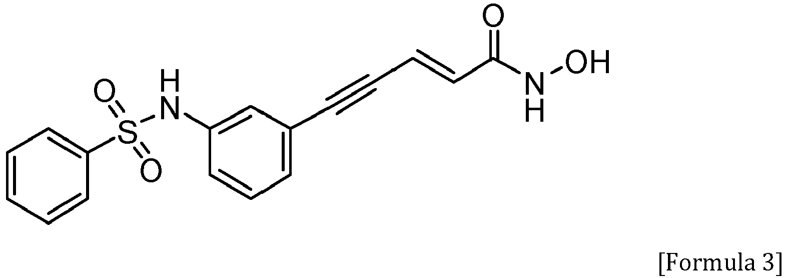

The invention also provides a method of treatment of a kidney disease comprising administering a therapeutically effective amount of Formula 1 as described above for the compound for use. In a particularly preferred embodiment, the invention provides a method of treatment of a kidney disease comprising administering a therapeutically effective amount of (2E)-N-hydroxy-3-[3-(phenylsulfamoyl)phenyl]prop-2-enamide, or a salt, hydrate, solvate, metabolite, or prodrug thereof. In another particularly preferred embodiment, the invention provides a method of treatment of a kidney disease comprising administering a therapeutically effective amount of 5-[3-(benzenesulfonamido)phenyl]-N-hydroxypent-2-en-4-ynamide, or a salt, hydrate, solvate, metabolite, or a prodrug thereof.

In another preferred embodiment, the compound for use of the invention is an analog of oxamflatin (Formula 3) or of a metabolite or prodrug thereof. In a particularly preferred embodiment, the compound for use of the invention is an analog of oxamflatin (Formula 3), in particular one selected from the group consisting of 3-Methanesulfonylamino-biphenyl-3-hydroxamic acid, 3-Methanesulfonylamino-biphenyl-4-hydroxamic acid, and (2*E*)(4*E*)-5-(3-Methanesulfonylaminophenyl) pentadienohydroxamic acid.

### Treatment

It is contemplated that the present invention is relevant to the treatment of a broad range of diseases affecting the kidney. In some embodiments, the compounds of the invention are used to treat chronic and/or acute kidney disease.

In one embodiment, the chronic kidney disease is selected from the group consisting of primary or secondary kidney disease. The term primary kidney disease refers to a kidney disease that has its origin in the kidney. Primary kidney diseases can be grouped into various categories, such as glomerulonephritis, tubulointerstitial disease, genetic kidney disease, and kidney cancer. In one embodiment of the invention, the compounds are used for the treatment and/or prevention of a primary kidney disease selected from the group consisting of glomerulonephritis, tubulointerstitial disease, genetic kidney disease, and kidney cancer. In a preferred embodiment of the invention, the compounds are used for the treatment and/or prevention of a primary kidney disease selected from the group consisting of glomerulonephritis, tubulointerstitial disease, and genetic kidney disease; most preferably glomerulonephritis.

Preferably, the glomerulonephritis is selected from the group consisting of primary focal and segmental glomerulosclerosis (also referred to herein as primary "FSGS"), genetic FSGS (for example FSGS caused by one or more mutations of one or more of the 60 known genes such as NPHS1, NPHS2, WT1, MAGi2, and/or APOL1, preferably APOL1), Alport syndrome, Fabry disease, Lupus nephritis, minimal change nephropathy, membranous glomerulonephritis, IgA glomerulonephritis, IgM glomerulonephritis, membranoproliferative glomerulonephritis, crescentic glomerulopathies (acute and chronic), age-related glomerulopathy (for example due to the loss of nephrons and/or podocytes), and cystic kidney disease. In a particularly preferred embodiment, the compounds are used in the treatment and/or prevention of a glomerulonephritis selected from the group consisting of primary FSGS, genetic FSGS, minimal change nephropathy, and age-related glomerulopathy.

Preferably, the tubulointerstitial disease is selected from the group consisting of interstitial nephritis, tubular acidosis, kidney stone-induced tubulointerstitial disease, urinary-tract infection-induced tubulointerstitial disease, and nephronophthisis. Preferably, the genetic kidney disease is a polycystic kidney disease.

The term secondary kidney disease refers to a kidney disease that has a systemic origin and did not originate in the kidney. Preferably the secondary kidney disease is selected from the group consisting of secondary FSGS, hypertension-induced nephropathy, diabetic nephropathy, minimal change disease, membranous nephropathy, a virus-induced kidney disease (for example HIV, SARS-CoV2, malaria, hepatitis-induced kidney disease), a systemic autoimmune disease (for example systemic lupus erythematosus), a drug-induced kidney disease (for example a NRSA, antibiotic, lithium, or inorganic salt such as gold and mercury-induced kidney disease), a tumor-induced kidney disease (for example a lung and/or colon tumor-induced kidney disease), immune complex-induced kidney disease, in the blood circulating antibody against e.g. phospholipase A₂, NELL-1, EXT1/2, Semaphorin 3B, HTRA1, PCDH7, a crescentic glomerulopathy, a toxin-induced kidney disease, a vascular and/or heart disease-induced kidney disease, and cystinosis. In a particularly preferred embodiment, the kidney disease is selected from the group consisting of secondary FSGS, hypertension-induced nephropathy, diabetic nephropathy, minimal change disease, and membranous nephropathy. Most preferably, the kidney disease is selected from the group consisting of secondary FSGS and minimal change disease.

The kidney disease treated according the invention is not associated with or the result of a peripheral T-cell lymphoma. The treatment according to the invention does not comprise the treatment of a peripheral T-cell lymphoma.

In some embodiments, the kidney disease is an acute kidney injury, preferably selected from the group consisting of an infection-induced acute kidney injury (for example a bacteria or virus-induced kidney injury), dehydration-induced acute kidney injury, drug- or drug treatment-induced acute kidney injury (such as antibiotic-induced kidney injury), an obstruction of urine flow-induced acute kidney injury (for example from one or more kidney stones), a surgery-induced acute kidney injury, and necrosis-induced acute kidney injury.

In some embodiments, the kidney disease is a kidney disease associated with podocyte injury and/or podocyte foot process effacement. In a preferred embodiment, the kidney disease is a podocytopathy; in particular a podocytopathy selected from the group consisting of diffuse mesangial sclerosis, focal segmental glomerulosclerosis, minimal change disease, and collapsing glomerulopathy. In another preferred embodiment, the kidney disease is a genetic kidney disease associated with one or more genes located in the podocyte, preferably selected from the group consisting of COQ2/6, PDSS2, ADCK4, MTT1, MT-ATP6, PTPRO, EMP2, APOL1, PODXL, SCARB2, ZMPSTE24, PMM2, DGKE, GAPVD1, ANKFY1, SPGL1, TBC1D8B, CLCN5, ACTN4, MYH9, INF2, MYOE1, MAGI2, TNS2, DLC1, CDK20, ITSN1, ITSN2, ANLN, ARGHGAP24, ARGH-DIA, KANK1/2/4, SYNPO, AVIL, KAT2B, KEOPS-complex, WT1, LMX1B, SMARCAL1, NUP85/93/107/133/160/205, XPO5, E2F3, NXF5, PAX2, LMNA, MAFB, WDR73, NPHS1/2, PLCE1, CD2AP, TRPC6, CRB2, FAT1, and/or KIRREL1/2; in particular one or more genes selected from the group consisting of NPHS1/2, WT1, MAGI2, and/or APOL1.

As used herein, "treatment" means any manner in which one or more symptoms associated with a disease are beneficially altered. Accordingly, the term includes healing or amelioration of a symptom or side effect of the disease or a decrease in the rate of advancement of the disorder.

The term "subject" is intended to include humans, primates, livestock animals (e.g. horses, camels, cattle, sheep, pigs and donkeys), laboratory test animals (e.g. mice, rats, rabbits, guinea pigs), companion animals (e.g. dogs, cats), captive wild animals (e.g. kangaroos, deer, foxes), poultry birds (e.g. chickens, ducks, bantams, pheasants), reptiles, and fish. In a preferred embodiment, the subject is a human or a laboratory test animal. In a particularly preferred embodiment the subject is a human.

### Formulation. Dosage, and Administration

The compounds of the present invention are formulated as a pharmaceutical composition and can be administered in any suitable manner. Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

These compositions may also contain adjuvants such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

The formulation comprises the compound of interest and may optionally comprise further pharmaceutically acceptable ingredients well known to those skilled in the art. These components include, but are not limited to, pharmaceutically acceptable carriers, diluents, excipients, adjuvants, buffers, preservatives, anti-oxidants, stabilizers, solubilizers, surfactants, and the like.

In some embodiments, the formulation may further comprise other active agents, for example, other therapeutic or prophylactic agents.

Pharmaceutical formulations may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Preferably, especially when the compound is belinostat or oxamflatin, it is prepared and administered in a form suitable for oral or parenteral administration, particularly preferred for intravenous administration. The pharmaceutical formulations may be prepared by any method known in the art of pharmacy, for example by bringing into association the active ingredient with the carrier(s) or excipient(s).

It should be understood that in addition to the ingredients particularly mentioned above, the formulations may include other agents conventional in the art having regard to the type of formulation in question, for example those suitable for oral administration may include flavoring agents.

As used herein the term "therapeutically effective amount" means an amount necessary to at least partly attain the desired response. A therapeutically effective amount of a compound or a pharmaceutically acceptable salt, solvate, hydrate, metabolite or prodrug thereof will depend upon a number of factors including, for example, the age and weight of the animal, the precise condition requiring treatment and its severity, the nature of the formulation, and the route of administration, and will ultimately be at the discretion of the attendant physician or veterinarian.

In embodiments wherein the compound for use is belinostat, it is preferred that belinostat is prepared and administered in a form suitable for oral or parenteral administration, particularly preferred for intravenous administration. In a preferred embodiment, belinostat is administered by infusion, in particular intravenous infusion.

Belinostat for injection is typically supplied as a sterile lyophilized yellow powder containing 500 mg belinostat as the active ingredient for reconstitution. Each vial also contains 1000 mg L-Arginine, USP as an inactive ingredient. Belinostat is intended for intravenous administration after reconstitution with 9 mL sterile water for injection, and the reconstituted solution is further diluted with 250 mL of sterile 0.9% sodium chloride injection prior to infusion. Its approved recommended dosage is 1000 mg/m² administered over 30 minutes by intravenous infusion once daily on days 1-5 of a 21 day cycle. Cycles can be repeated every 21 days until disease progression or unacceptable toxicity. While typically, belinostat is administered intravenously over 30 minutes, if infusion site pain or other symptoms potentially attributable to the infusion occur, the infusion time may be extended to 45 minutes.

In one embodiment, belinostat is administered at a therapeutically effective dose intravenously, preferably as a bolus, by a 30- to 45-minute intravenous infusion on five consecutive days. In a particularly preferred embodiment, belinostat is administered at about 1000 mg/m² administered over a 30- to 45-minute intravenous infusion once daily on days 1-5 of a 21 day cycle, wherein optionally cycles can be repeated every 21 days until disease progression or unacceptable toxicity.

In some embodiments, belinostat is administered as a prolonged continuous infusion (e.g. prolonged continuous intravenous infusion). In a preferred embodiment, the prolonged continuous infusion is for a period of at least about 48 hours, for example, a period of from 48 to 72 hours, or a period of from 48 to 96 hours. In one embodiment, belinostat is administered as a prolonged continuous infusion (e.g. prolonged continuous intravenous infusion) for a period of at least 72 hours to 96 hours.

In other embodiments, the belinostat may be administered in an oral form, in particular by administering an amorphous solid dispersion of belinostat comprising (a) belinostat or a pharmaceutically acceptable salt, solvate or ester thereof, and (b) at least one non-ionizable (neutral) non-cellulosic polymer, such as a polyvinyl lactam polymer according to WO2019002614 A1. In other embodiments, belinostat can be administered in a crystalline form, in particular in the form of a crystalline acetone solvate of belinostat according to US2021292276 A1.

It will be appreciated by one of skill in the art that appropriate dosages of belinostat can vary between patients. Determining the optimal dosage will generally involve the balancing of the level of therapeutic benefit against safety risks. In some embodiments, the dosage of belinostat when treating humans is from 5 to 2500 mg/m²/d; more preferably from 10 to 1000 mg/m²/d or from 50 to 500 mg/m²/d; most preferably from 100 to 500 mg/m²/d. In another preferred embodiment, the dosage of belinostat when treating humans is from 100 to 2500 mg/m²/d; more preferably from 500 to 1500 mg/m²/d; most preferably from 900 to 1000 mg/m²/d. When treating non-human animals, the dosage will be adjusted accordingly. In non-human animals, especially in rodents such as rats and mice, belinostat is preferably administered in a dose of from 1 to 500 mg/kg/d, preferably from between 5 and 100 mg/kg/d, more preferably from 25 to 75 mg/kg/d. Where belinostat is provided as a salt, hydrate, solvate, metabolite or prodrug, the amount administered is calculated on the basis of the parent compound and so the actual weight to be used is adjusted proportionately.

The above-described aspects of the invention will be further described in the following examples and figures. These are strictly illustrative of particularly preferred embodiments of the invention. The invention should not be construed, however, to be limited to such embodiments.

### EXAMPLES

### Example 1

### Zebrafish strains and husbandry

The zebrafish larva has proven to be an elegant option for drug screens since it combines a high experimental throughput with the advantages of being an *in vivo* model. Optical transparency, expression of fluorescence reporters via genetic engineering, easy and economic maintenance and a 70% genetic homology to humans provide decisive experimental advantages (Howe, K., Clark, M., Torroja, C. et al. (2013) The zebrafish reference genome sequence and its relationship to the human genome. Nature 496, 498-503). A high conservation of the renal filtration apparatus including a strong resemblance of the glomerular filtration barrier make zebrafish larvae very suitable for glomerular research.

Zebrafish stocks were maintained as previously described (Schindler, Maximilian, et al. "Adriamycin does not damage podocytes of zebrafish larvae." Plos one 15.11 (2020): e0242436). Several groups of two males and two females were permanently put together as mating pairs for the screening experiments. These groups were always composed of two individuals of the following zebrafish strains: Tg(-*3.5fabp10a:*gc-eGFP); *mitfaw2lw2*;*mpv17a9l*a9 and Tg(-3.5fabp10a:gc-eGFP);Tg(*nphs2*:GAL4-VP16); Tg(*UAS*:Eco.nfsB-mCherry); *mitfaw2*/w2; *mpv17a9*/a9 (Xie, Jing, et al. "A novel transgenic zebrafish model for blood-brain and blood-retinal barrier development." BMC developmental biology 10.1 (2010): 1-14; Zhou, Weibin, and Friedhelm Hildebrandt. "Inducible podocyte injury and proteinuria in transgenic zebrafish." Journal of the American Society of Nephrology 23.6 (2012): 1039-1047; Siegerist, Florian, et al. "Acute podocyte injury is not a stimulus for podocytes to migrate along the glomerular basement membrane in zebrafish larvae." Scientific reports 7.1 (2017): 1-12). Larvae from these groups were raised under standard conditions until 4 dpf and transgene expression was checked with a SMZ 18 fluorescence stereomicroscope (Nikon GMBH, Düsseldorf, Germany) under 0.1 mg/ml tricaine anesthesia (MS-222, #E10521, Merck KGaA, Darmstadt, Germany). All experiments adhered with the German animal protection law and were overseen and approved by the "Landesamt fur Landwirtschaft, Lebensmittelsicherheit und Fischerei, Rostock" (LALLF M-V) of the federal state of Mecklenburg - Western Pomerania.

### Induction of podocyte injury and drug treatment

Groups of 12 larvae from one clutch were distributed into a 24-well plate (#662160 Greiner, Frickenhausen, Germany). For each clutch, 12 larvae were treated with 0.2% DMSO in E3 as negative control and 12 larvae were treated with 80 µM Metronidazole (MTZ) in DMSO in E3 as injury control and comparison group. The Fraunhofer ScreeningPort IME provided an epigenetic drug library which was used for this screening (Table 1). Compounds from this library were dissolved in MTZ solution in order to perform a co-treatment of larvae with 80 µM MTZ + 100 µM compound in DMSO in E3. The treatment volume was 400 µl for each well with 12 larvae, the wells were sealed with Adhesive Clear PCR Seal (#600208, Biozym Scientific GMBH, Hessisch-Oldendorf) in order to prevent evaporation and plates were incubated at 28.5°C, wrapped in aluminum foil to prevent effects of light. After 24 hours, compounds were washed out in three changes of 0.5 x E3.

### Preparation for imaging

After washout at 5 dpf, E3 in the 12-well plate was replaced with E3 containing 0.4 mg/ml tricaine. Larvae were then individually transferred into wells of a 96-well plate (#644101, Greiner) which was prepared with custom agarose molds. For the molds, 45 µl of 0.7% boiling hot agarose (Biozym LE agarose) were transferred with a multichannel pipette into each well. After 3 min cooldown, a custom 3D printed orientation tool (Wittbrodt, Jonas N., Urban Liebel, and Jochen Gehrig. "Generation of orientation tools for automated zebrafish screening assays using desktop 3D printing." BMC biotechnology 14.1 (2014): 1-6) was inserted into the agarose which produced cavities for orientation. Each larva was transferred with 100 µl tricaine containing E3 into the single wells of the 96-well plate (Row A was always 12x DMSO, Row B was always 12x MTZ, Row C-H were compounds + MTZ) and was then oriented laterally in the molds with a short piece of fishing line glued to a bend injection needle under the stereomicroscope manually with special attention to a flat caudal region.

### Imaging

Image acquisition for the screening was performed with an Imaging Machine (ACQUIFER Imaging GmbH, Heidelberg, Germany). This high-content screening device possesses a white LED array for brightfield images, a LED for fluorescence excitation, a sCMOS (2048 × 2048 pixel) camera, a temperature-controlled incubation chamber (set to 28.5 °C during acquisition), a stationary plate holder and moving optics. Overview images of each laterally oriented larva were obtained with a 2x objective (40% LED intensity, 30 ms integration). For each larva, a region of interest (ROI) at the tail region caudal of the cloaca was selected with the Acquifer PlateViewer (V1.7.1, ACQUIFER) for the 10x objective in order to image the blood flow and vasculature. After creating custom modificated imaging scripts via FIJI (Schindelin, Johannes, et al. "Fiji: an open-source platform for biological-image analysis." Nature methods 9.7 (2012): 676-682), 10 consecutive brightfield frames at a framerate of 33 Hz were acquired (10x objective, 100% LED intensity, 30 ms integration) followed by one frame at 470 nm (10x objective, 50% LED intensity, 50 ms integration). Next, the ROI of each larva was dragged to the glomerular region in the PlateViewer, scripts were centered and one frame of each larval glomerulus at 555 nm was acquired (10x objective, 40% LED intensity, 40 ms integration). The focal plane for both acquisitions was detected with a built-in two-step software autofocus (10x objective, 2x2 binning, 10% LED intensity, 10 ms integration, first step: 31 slices with 50 µm distance, second step: 10 slices with 10 µm slice distance, brightfield for the caudal vasculature, 555 nm for glomerular mCherry).

Following image acquisition, tricaine-containing E3 was washed out two times of each well and replaced by 150 µl E3. The 96-well plate was again sealed and incubated for another 24 h at 28.5°C. At 6 dpf, larvae were again anesthetized with 0.4 mg tricaine, oriented laterally and imaged again in the same way as mentioned before. In this way, two values for the vascular eGFP fluorescence and two values for the glomerular mCherry fluorescence were obtained for each individual larva.

### Data storage and processing

An Acquifer HIVE (ACQUIFER) was used for data storage and processing. Custom written FIJI macros were created and used for image analysis. The macros were written so that a full folder of raw image data of one 96-well plate could be dragged into and directly processed by FIJI.

### Image analysis

### Vascular fluorescence - proteinuria

Vascular eGFP fluorescence was segmented and measured fully automatic via a custom FIJI macro. For each larva, 10 brightfield slices and one slice at 470 nm were acquired at the caudal region. The macro fuses the brightfield slices to a short movie in which the blood flow is clearly observable due to the movement of erythrocytes. After blurring and background subtraction, the standard deviation of this movie is created. This standard deviation shows all areas in which pixel changes occur, which are, in this case, circulating erythrocytes. Subsequent thresholding and size restriction allows the creation of ROIs which distinctively detect areas of blood flow. These ROIs are then used as segmentation masks for the 470 nm image in which the mean fluorescence is measured exclusively in the blood vessels due to prior blood flow detection.

### Glomerular fluorescence - degree of podocyte depletion

Fluorescence of the nphs2-driven podocyte mCherry was segmented and measured fully automatic via FIJI as well. For each larva and each image obtained with 555 nm, the center of mass (x and y coordinate of the brightness-weighted average of all pixel) is determined which is in this case the center of the pronephric glomerulus. A circular custom ROI is created and gets automatically drawn to the center. The ROI for the mCherry signal is always of the same size (70688 pixel with 0.65 µm/pixel ratio) and the mean fluorescence intensity in this ROI is measured for each larva and both time points.

### Data analysis

Both macros create excel files with all necessary data. The macro for the vascular fluorescence additionally creates a subfolder with images of the segmentation masks for the possibility to check the segmentation process. By a build-in exclusion algorithm, larvae in which no vascular blood flow is detected, are not analyzed and values of these larvae will not appear in the excel files but in a text file. Values from these larvae will then also be excluded from the excel files of the glomerular measurement.

For both readouts and each larva there is one mean fluorescence value at 5 dpf right after the treatment and one at 6 dpf. Vascular fluorescence and glomerular fluorescence ratios were calculated for each larva and gaussian distribution was checked with a Kolmogorov-Smirnov test for each readout and each separate plate. Ratios of treatment groups were subsequently analyzed by either ANOVA followed by a Dunnet's multiple comparison test or by a Kruskal-Wallis test followed by Dunn's multiple comparison. The MTZ group on every plate served as a control group, *p* < 0.05 was regarded as statistically significant. Data analysis and graphs were created with GraphPad Prism 9.1.2 (GraphPad Software, San Diego, California USA). Volcano plots were created using VolcaNoseR (10.1101/2020.05.07.082263).

In order to cover two central aspects for the integrity of the larval glomerular filtration barrier, we used the Tg(-*3.5fabp10a:*gc-eGFP); Tg(*nphs2*:GAL4-VP16); Tg(*UAS:*Eco.nfsB-mCherry); *mitfaw2*/*w2*; *mpv17a9*/a9 strain for all screening experiments. This strain expresses a circulating fusion protein of the vitamin D-binding protein and eGFP (VDP-eGFP) with a size of 78 kDa, which under healthy steady-state conditions cannot pass the glomerular filtration barrier. Impairment of the glomerular filtration barrier directly results in clearance of VDP-eGFP from the vasculature and can be used as a surrogate parameter for proteinuria. Additionally, podocyte-specific expression of the bacterial nitroreductase and mCherry allows microscopic *in vivo* assessment of podocyte presence and podocyte injury induction by immersion treatment (Siegerist, Florian, et al. "Acute podocyte injury is not a stimulus for podocytes to migrate along the glomerular basement membrane in zebrafish larvae." Scientific reports 7.1 (2017): 1-12).

The inventors found that a lateral orientation of the larvae gives perfect microscopic access for the Acquifer Imaging Machine to both readouts, the vascular eGFP fluorescence (degree of Proteinuria) at the tail region and the podocyte mCherry signal (degree of podocyte depletion). Custom agarose molds (Wittbrodt, Jonas N., Urban Liebel, and Jochen Gehrig. "Generation of orientation tools for automated zebrafish screening assays using desktop 3D printing." BMC biotechnology 14.1 (2014): 1-6) were used to generate cavities that allow a swift lateral orientation of 96 larvae per plate.

For the vascular eGFP fluorescence, a region of interest (ROI) was selected with the 10x objective on the basis of an overview image (2x objective) of each larva in a 96-well plate. Using the standard deviation of the pixel change (highest in vasculature with moving blood cells), we were able to reliably and automatically segment the vasculature in zebrafish larvae via FIJI. The custom FIJI analysis script also detects larvae with no blood flow in the caudal vasculature and eliminates these larvae from analysis. This ensures that only viable larvae become part of the evaluation.

For automated segmentation of the mCherry signal in podocytes, a different approach was developed. The segmentation in the brightfield channel is not possible for the glomerulus due to a lack of contrast or pixel value change in the target structure. We directly used the mCherry fluorescence image as a segmentation template and were able to automatically and reliably detect podocytes and measure the mean fluorescence intensity in a defined area.

Variation of transgenic reporter expression levels can vary over clutches of embryos from different founders and therefore possibly increasing heterogeneity in datasets obtained over several weeks. To circumnavigate this, the workflow of this new assay performs two measurements of both readouts for each larva, one at 5 dpf and one at 6 dpf. This generates robust fluorescence ratios for the degree of proteinuria and podocyte condition.

Adding metronidazole (MTZ) to the larval medium causes DNA crosslinking in cells that express the nitroreductase. By podocyte-specific nitroreductase expression, this causes an injury exclusively in podocytes. It has been reported that the degree of injury is dependent on MTZ concentration and exposure time (Zhou, Weibin, and Friedhelm Hildebrandt. "Inducible podocyte injury and proteinuria in transgenic zebrafish." Journal of the American Society of Nephrology 23.6 (2012): 1039-1047). The inventors' previous work has shown that a treatment of Tg(*nphs2:*GAL4); Tg(*UAS:Eco.nfsb*-mCherry) larvae at 4 dpf for 48 hours with 80 µM MTZ resulted in a phenotype that resembles mammalian FSGS at 8 dpf (Hansen, Kerrin Ursula Ingeborg, et al. "Prolonged podocyte depletion in larval zebrafish resembles mammalian focal and segmental glomerulosclerosis." The FASEB Journal 34.12 (2020): 15961-15974). In order to avoid feeding and to accelerate the protocol, the MTZ concentration was recalibrated on the Tg(-*3.5fabp10a:*gc-eGFP); Tg(*nphs2*:GAL4-VP16); Tg(*UAS*:Eco.nfsB-mCherry); *mitfaw2*/*w2*; *mpv17a9*/a9 screening strain.

Larvae were treated with 80 µM, 160 µM and 320 µM MTZ for 24 hours. After washout at 5 dpf there was no difference between the DMSO control and the 80 µM group in the mCherry signal. Treatment with 160 µM and 320 µM resulted in a loss of glomerular mCherry and an accumulation of the fluorescence protein in the proximal convoluted tubules.

Imaging of the same larvae after another 24 hours at 6 dpf revealed a loss of mCherry fluorescence in the 80 µM MTZ group compared to the DMSO control. MTZ-induced glomerular injury in the 160 µM and 320 µM lead to a further reduction of mCherry signal intensities, close to a complete loss of podocytes. Podocyte injury or loss leads to proteinuria, in the case of our screening strain to a quantifiable leakage of the VDP-eGFP. After washout of MTZ at 5 dpf, the eGFP signal was not reduced in the 80 µM MTZ group compared to DMSO. Treatment with 160 µM MTZ for 24 h caused a reduction of the vascular eGFP signal and a visible uptake of eGFP in the proximal convoluted tubules. Treatment with 320 µM resulted in a strong glomerular leakage of the VDP-eGFP. At 6 dpf, a clear vascular loss and tubular uptake of the VDP-eGFP was detected in 80 µM MTZ treated larvae compared to the DMSO control. The glomerular leakage in the 160 µM MTZ and 320 µM MTZ group progressed to a state in which a clear vascular signal could not be detected anymore at 6 dpf.

Edema formation is a hallmark of a disrupted filtration barrier in zebrafish (Huang, Jianmin, et al. "A zebrafish model of conditional targeted podocyte ablation and regeneration." Kidney international 83.6 (2013): 1193-1200). Exposure of larvae to 80 µM MTZ for 24 hours from 4-5 dpf induced no edema formation at 5 dpf but slight edema formation at 6 dpf. Slight edema were visible after treatment with 160 µM at 5 dpf, which progressed to severe edema at 6 dpf. Larvae treated with 320 µM MTZ developed severe edema at 5 dpf which even progressed until 6 dpf. Quantifications of the fluorescence intensity ratios by our new screening setup confirmed the prior results. A significant loss of glomerular mCherry and vascular eGFP could be detected in all three MTZ treatment groups by our new assay from 5 to 6 dpf. These results clearly confirm the reliability of this screening method. A treatment with 80 µM MTZ for 24 hours resulted in a quantifiable proteinuric phenotype and podocyte depletion in the timeframe of our screening setup and will be used for all further experiments.

### Example 2

After successful establishment of a workflow and injury induction, a drug library consisting of 138 compounds was used to identify drugs that potentially hamper or prevent podocyte injury and subsequent proteinuria.

12 larvae were treated per compound in conjunction with MTZ from 4 to 5 dpf. In order to maintain high data consistency and to have an inter-run calibrator for each individual 96-well plate, 12 larvae from every clutch were DMSO treated, 12 larvae received MTZ only and the next groups of 12 larvae were exposed to MTZ plus 100 µM compound. The fluorescence ratios of the treatment groups served as statistical input and were compared to the MTZ podocyte injury group. A total of 41 96-well plates, each with 24 internal control larvae, were screened.

Out of 138 compounds, a total of 36 compounds had a lethal effect on zebrafish larvae within the 24 hours treatment from 4 to 5 dpf. Out of the 102 remaining compounds tested, two compounds were found to be podocyte-protective in this injury model: belinostat and oxamflatin.

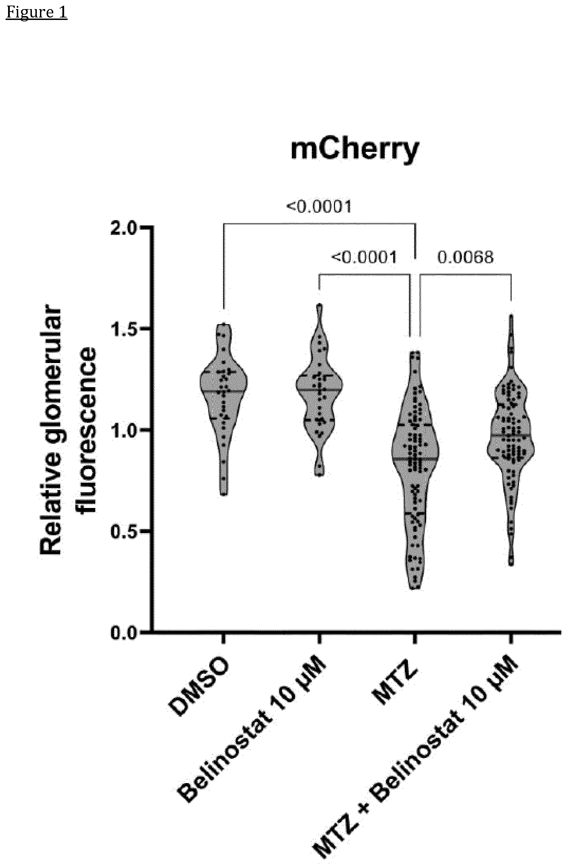

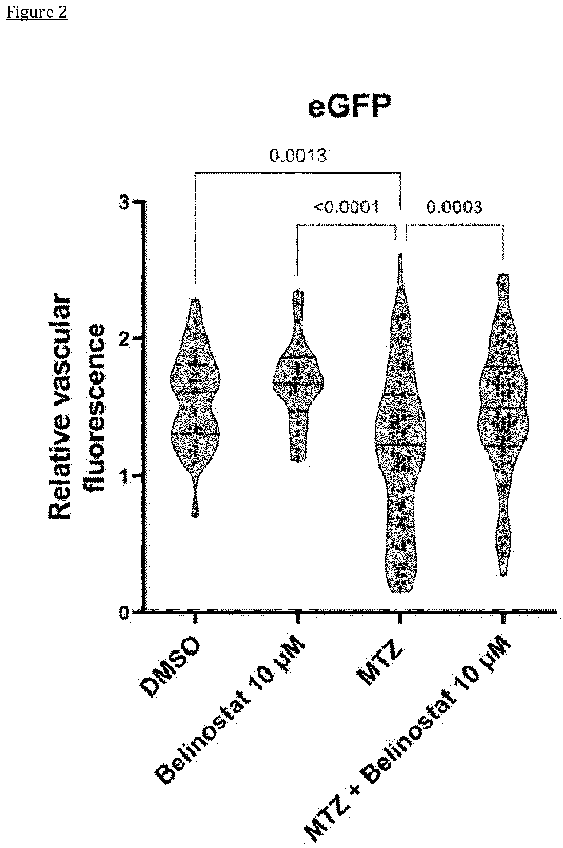

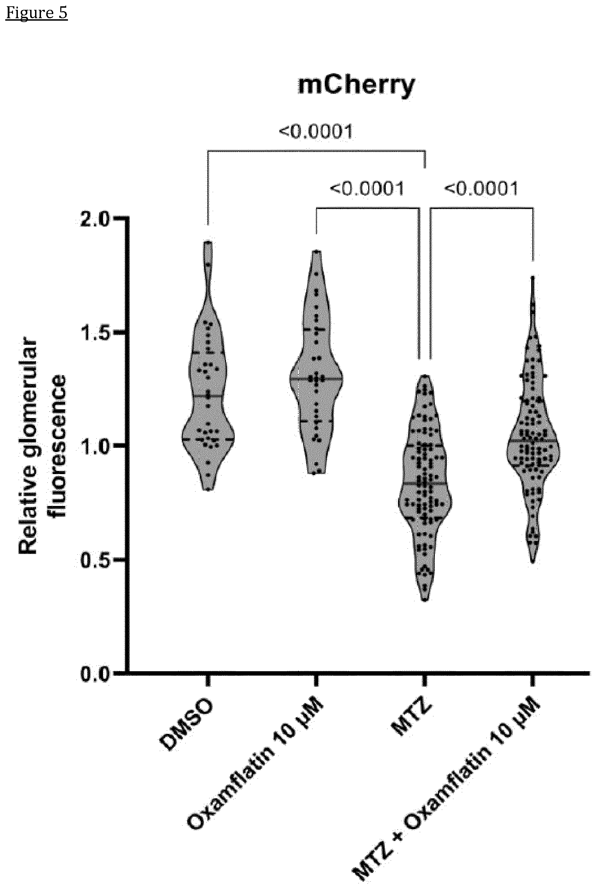

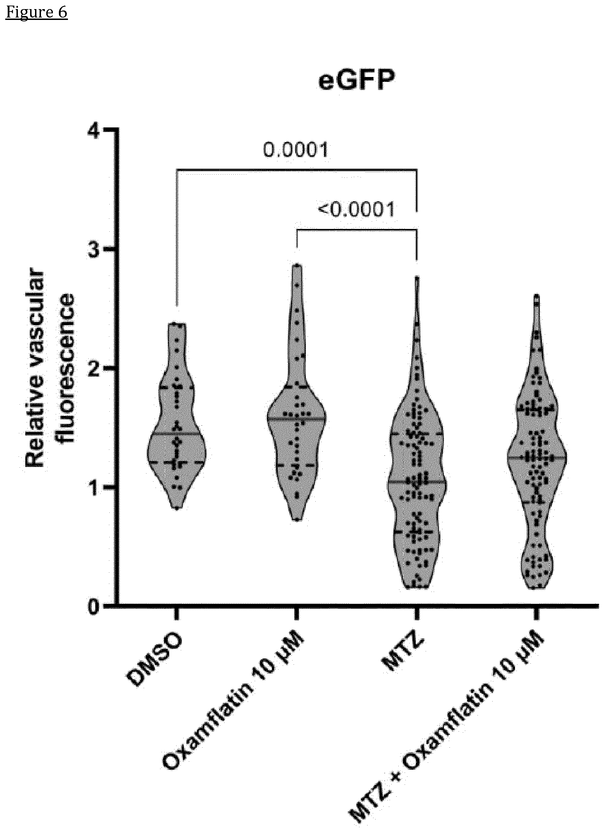

Transgene expression of nphs2:ntr-mCherry in podocytes (Figure 1) and the VDP-eGFP in the vasculature (Figure 2) was verified with a stereomicroscope at 4 dpf as described in Example 1. Larvae from one clutch were distributed in groups of 12 in 24-well plates for treatments. After 24 hours, all compounds were washed out and the larvae are placed individually into a 96-well plate. Following lateral orientation, images of vascular and glomerular fluorescence were acquired at 5 dpf. After another 24 hours, the vascular and the glomerular fluorescence was acquired again of the exact same larvae. The fluorescence ratios of both readouts (fluorescence value at 6 dpf / fluorescence value at 5 dpf) for each individual larva were used to depict the fluorescence intensity changes after treatment between 5 and 6 dpf and serve as input for the statistics. The fluorescence ratios in the belinostat treatment example are shown in Figures 1 and 2 and those in the oxamflatin treatment example are shown in Figures 5 and 6.

Treatment with 10 µM belinostat had no harmful effect on the glomerular filtration barrier or podocyte vitality. The podocyte injury by MTZ caused a significantly higher renal leakage of eGFP from the vasculature and a loss of podocyte vitality. Addition of 10 µM belinostat to the 80 µM MTZ solution could prevent the renal leakage of the 78 kDa VDP-eGFP through the glomerular filtration barrier (Figure 2). This result was corroborated by a significantly reduced loss of podocyte mCherry fluorescence (Figure 1) in the co-treatment group of 10 µM belinostat and 80 µM MTZ in comparison to MTZ (DMSO n=30, belinostat n=30, MTZ n=89, MTZ+belinostat n=85).

Similar results were shown after analogous treatment with 10 µM oxamflatin (Figures 5 and 6).

### Example 3

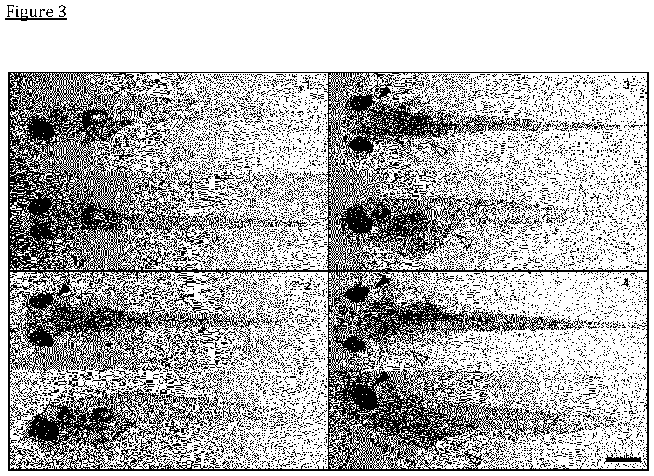

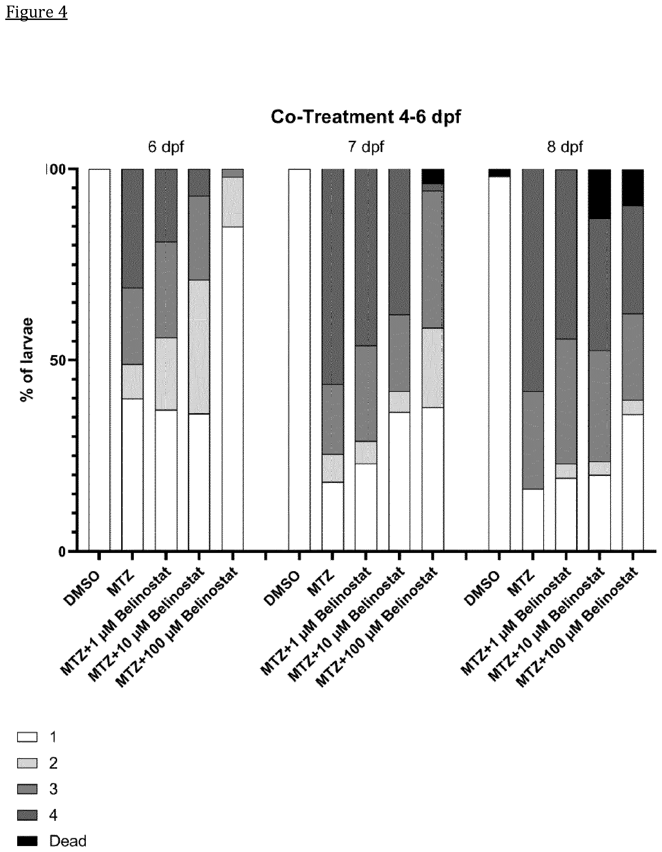

A co-treatment with MTZ and belinostat at 10 µM resulted in a significant reduction of proteinuria and podocyte depletion. To corroborate the results of Example 2 for belinostat, a serial dilution of belinostat was used in a co-treatment with 80 µM MTZ on Tg(*nphs2*:GAL4-VP16); Tg(*UAS*:Eco.nfsB-mCherry); *mitfaw2*/*w2*; *mpv17a9*/a9 larvae for 48 hours from 4 to 6 dpf. The results are depicted in Figure 4. A treatment for 48 hours from 4 to 6 dpf with MTZ induces edema formation of four different severities in this strain, scaling from type 1= no edema to type 4= severe edema (Figure 3). Edema severity of larvae treated with DMSO, MTZ and MTZ plus 1 µM, 10 µM or 100 µM belinostat was categorized at 6, 7 and 8 dpf (Figure 4). After washout at 6 dpf, all three treatment groups with MTZ + belinostat showed a reduced number of larvae with type 3 and 4 edema in a dose-dependent manner. Co-treatment with MTZ and 100 µM belinostat induced edema formation in 15% of larvae with 0% type 4 edema in contrast to 60% edema with 31% type 4 edema in the MTZ group. At 7 dpf, progression of edema formation was decelerated in all three groups for all three types of edema that received belinostat, again in a dose-dependent manner. MTZ caused type 4 edema in 56.4% of larvae at 7 dpf, contrary to 1.9% edema in the MTZ + 100 µM belinostat group. The delay in edema formation in the belinostat treatment groups stayed consistent at 8 dpf. 83,6% of larvae treated with MTZ developed edema with 58.2% showing type 4 edema. Treatment with 1 µM belinostat + MTZ resulted in 80.8% edema formation with 44.2% being type 4 edema. 10 µM belinostat + MTZ induced edema 80% of larvae with 34.5% type 4 edema. Co-treatment of MTZ with 100 µM belinostat caused edema formation in 64.2% of larvae with 28.3% type 4 edema (Figure 4). These results clearly confirm the protective effect of belinostat in a dose-dependent manner on podocytes in this injury model.

### BRIEF DESCRIPTION OF THE DRAWINGS

Figure 1 depicts the mCherry fluorescence signal measured in the control DMSO group (n=30), the injury 80 µM MTZ group (n=89), the 10 µM belinostat group without injury (n=30), and the co-treatment group of 10 µM belinostat and 80 µM MTZ (n=85) described in Example 2. Data distribution was checked via Kolmogorov-Smirnov test and significance was determined by ordinary one-way ANOVA, followed by Dunnet's multiple comparison test. The MTZ group served as control, p≤0.05 was considered statistically significant.
Figure 2 depicts the eGFP fluorescence signal measured in the control DMSO group (n=30), the injury 80 µM MTZ group (n=89), the 10 µM belinostat group without injury (n=30), and the co-treatment group of 10 µM belinostat and 80 µM MTZ (n=85) described in Example 2. Data distribution was checked via a Kruskal-Wallis test followed by Dunns's multiple comparison. The MTZ group served as control, p≤0.05 was considered statistically significant.
Figure 3 depicts the different degrees of podocyte injury progression: 1: no edema, 2: slight edema, 3: moderate edema, 4: severe edema. Black arrowheads show periocular and blank arrowheads depict abdominal edema. Scale bar: 500 µm.
Figure 4 depicts the results of Example 3, measuring the edema progression in larvae that were treated with different concentrations of belinostat in conjunction with MTZ. Edema progression was measured according to the categories of in larvae until 8 dpf following MTZ injury with various concentration of belinostat (DMSO n= 52, MTZ n=55, MTZ+1 µM belinostat n=52, MTZ+10 µM belinostat n=55, MTZ+100 µM belinostat n=53).
Figure 5 depicts the mCherry fluorescence signal measured in the control DMSO group (n=32), the injury 80 µM MTZ group (n=96), the 10 µM oxamflatin group without injury (n=32), and the co-treatment group of 10 µM oxamflatin and 80 µM MTZ (n=96) described in Example 2. Data distribution was checked via Kolmogorov-Smirnov test and significance was determined by ordinary one-way ANOVA, followed by Dunnet's multiple comparison test. The MTZ group served as control, p≤0.05 was considered statistically significant.
Figure 6 depicts the eGFP fluorescence signal measured in the control DMSO group (n=32), the injury 80 µM MTZ group (n=96), the 10 µM oxamflatin group without injury (n=32), and the co-treatment group of 10 µM oxamflatin and 80 µM MTZ (n=96) described in Example 2. Data distribution was checked via a Kruskal-Wallis test followed by Dunns's multiple comparison. The MTZ group served as control, p≤0.05 was considered statistically significant.

## Claims

1. A compound for use in the treatment and/or prevention of a kidney disease, wherein the compound has the formula 1: 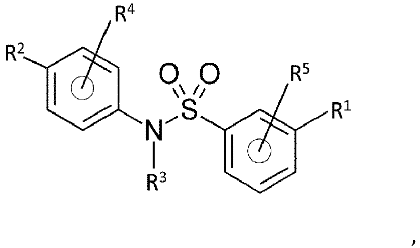
or a salt, hydrate, solvate, metabolite, or prodrug thereof;
wherein R¹ is selected from the group consisting of hydrogen, CH=CH-CH(O)NHOH, (CH₂)₂-C(O)NHOH, and C≡C-CH(O)NHOH;
R₂ is selected from the group consisting of hydrogen, (CH₂)₂-CH₂ and C=C-CH=CH-CH(O)NHOH;
R₃ is selected from the group consisting of H and C₁-C₆-alkyl; and
R₄ and R₅ are independently selected from the group consisting of hydrogen, halogen, C₁-C₆ alkyl, CN, and CF₃.

2. The compound for use according to claim 1, wherein R₁ is CH=CH-C(O)NHOH and R₂₋₅ are H, in particular wherein R₁ is (*E*)-CH=CH-C(O)NHOH and R₂₋₅ are H.

3. The compound for use according to claim 1, wherein R₁ and R₃₋₅ are H, and wherein R₂ is C≡C-CH=CH-C(O)NHOH, in particular C≡C-(*E*)-CH=CH-C(O)NHOH.

4. The compound for use according to any one of claims 1 to 3, wherein the compound is (2E)-N-hydroxy-3-[3-(phenylsulfamoyl)phenyl]prop-2-enamide or 5-[3-(benzenesulfonamido)phenyl]-N-hydroxypent-2-en-4-ynamide; or a salt, hydrate, solvate, metabolite, or prodrug thereof.

5. The compound for use according to claim 4, wherein the compound is a metabolite of (2E)-*N*-hydroxy-3-[3-(phenylsulfamoyl)phenyl]prop-2-enamide and wherein the metabolite is selected from the group consisting of belinostat amide, belinostat acid, methyl belinostat, belinostat gluconoride, 3-(anilinosulfonyl)-benzenecarboxylic acid (3-ASBA), and belinostatglucoside.

6. The compound for use according to claim 4, wherein the compound is a prodrug of (2E)-N-hydroxy-3-[3-(phenylsulfamoyl)phenyl]prop-2-enamide and wherein the prodrug is selected from the group consisting of ZL277 (Formula 2-4), ZL277-B(OH)₂-452 (Formula 2-5), and ZL277-OH-424 (Formula 2-6).

7. The compound for use according to any one of the preceding claims, wherein the compound is administered intravenously or orally, preferably intravenously.

8. The compound for use according to any of the preceding claims, wherein the compound is administered in a dosage of from 1 to 2500 mg/m²/d; more preferably from 5 to 2500 mg/m²/d, 10 to 1000 mg/m²/d, from 50 to 500 mg/m²/d, or from 100 to 500 mg/m²/d; most preferably from 100 to 500 mg/m²/d.

9. The compound for use according to any one of the preceding claims, wherein the kidney disease is an acute or chronic kidney disease.

10. The compound for use according to any one of the preceding claims, wherein the kidney disease is a kidney disease associated with podocyte injury and/or podocyte foot process effacement.

11. The compound for use according to any one of the preceding claims, wherein the kidney disease is a podocytopathy; in particular a podocytopathy selected from the group consisting of diffuse mesangial sclerosis, focal segmental glomerulosclerosis, minimal change disease, and collapsing glomerulopathy.

12. The compound for use according to any one of the preceding claims, wherein the kidney disease is a genetic kidney disease associated with one or more genes located in the podocyte.

13. The compound for use according to any one of the preceding claims, wherein the kidney disease is selected from the group consisting of secondary focal and segmental glomerulosclerosis, hypertension-induced nephropathy, diabetic nephropathy, minimal change disease, and membranous nephropathy.

14. The compound for use according to to any one of the preceding claims, wherein the kidney disease is selected from the group consisting of diabetic nephropathy, secondary focal and segmental glomerulosclerosis, and minimal change disease.

15. The compound for use according to any one of the preceding claims, wherein the compound is administered to a human or non-human animal, in particular to a human.
